# EUROPEAN PATENT APPLICATION

(11) **EP 2 338 567 A1**
(43) Date of publication of application: **29.06.2011**
(21) Application number: 09815699.5
(22) Date of filing: 11.09.2009
(51) Int. Cl.: A61N 2/02, A61N 5/00, A61N 1/00

(54) **ELECTROIMMUNISATION CHAMBER**

(30) Priority: 25.09.2008 ES 200802727
(71) Applicant: González Armas, Ernesto Miguel, 38760 Isla de La Palma (Santa Cruz de Tenerife) (ES); Rodríguez Martínez, Luis Gilberto, 38760 Isla de La Palma (Santa Cruz de Tenerife) (ES)
(72) Inventor: González Armas, Ernesto Miguel, 38760 Isla de La Palma (Santa Cruz de Tenerife) (ES); Rodríguez Martínez, Luis Gilberto, 38760 Isla de La Palma (Santa Cruz de Tenerife) (ES)
(86) International application number: PCT/ES2009/000449
(87) International publication number: WO 2010/034854

(57) **Abstract**

It consists of a horizontal space designed for treatment of patients with HIV, who place themselves in supine lying position, and the upper part of which has a cover with adjustable height with a magnetic pulse generator. The patient rest area also contains fixed electrodes in the lower part and variable electrodes in the upper patient area, which generate electrical impulses that supplement the magnetic pulses. The mechanisms for control and regulation of the chamber ensure dosing of electro-magnetic energy that acts on the ionic channels of the GP120 protein that is present in HIV. These channels open and lose the necessary energy used for interacting with the CD4 receptors, which are marking molecules on the cell surface, which recognise certain antibodies.

## Description

### OBJECT OF THE INVENTION

The invention patent that this statement refers to aims to guarantee exclusive use and ownership nationally of a therapeutic chamber with a system of devices (magnetic pulse generator and electrical impulse generator) intended for treatment of patients diagnosed with Human Immunodeficiency Virus (HIV).

### FIELD OF THE INVENTION

The application of this invention is in electrotherapy and magnetic therapy A61 N, particularly for electrotherapy A61 N 5/00 and use of magnetic fields A61 2/02.

This invention has an important aim, which is to act simultaneously on an individual diagnosed with HIV, provoking an irreversible alteration in the bioelectric activity that produces the protein Gp120 presented by this virus, invalidating its functions and making replication of the virus impossible, making the HIV vulnerable to the defence systems of the organism.

### ANTECEDENTS OF THE INVENTION.

Electro-immunisation is a technique with a solid basis in electromedicine, which makes use of important findings and results reached in genetic engineering.

As natural resources are applied in electro-immunisation it is considered a form of alternative medicine and it is carried out using modern technology, of course, so the technical infrastructure makes up a system of specific equipment for application.

No absolute conclusions have yet been reached, but there are currently very positive results with unimaginable scope for improving people's standard of living at a reduced cost.

Since the start of the 90's there have been some very similar investigations on the subject in the United States, but these were carried out under the constant vigilance of some pharmaceutical companies, which incorrectly considered that the positive results in this line of medical investigation constituted a threat to their market security.

At this time the investigations were in line with current electro-immunisation techniques, but the limiting factors, genetic characteristics and special properties of the materials were still not known.

### DESCRIPTION OF THE INVENTION.

The invention consists of a chamber for treatment of patients, combining a magnetic pulse generator and an electric impulse generator.

The magnetic pulse generator consists of a type of horizontal cuboidal cover made of insulating material with variable thickness and adjustable height. Inside the upper cover of the chamber there are electric coils fed by the discharges from a bank of capacitors that are in turn fed by a DC power supply.

The electric impulse generator consists of a base fixed to the patient's bed and two bars that can be adjusted to fit the height of the patient, to which are connected two insulated electrodes that cause controlled electrical stimulation in the organism of the patient.

### DESCRIPTION OF THE DRAWINGS.

For a better understanding of the scope of this invention, it will be described using the attached drawings, which show a preferred design for the invention. These drawings are provided as an example, and are not limiting in character.
Fig. 1 shows a schematic view of the invention with a recumbent patient,
Fig. 2 shows a view of the bed where the patient is placed,
Fig. 3 shows a circuit diagram of the electro-magnetic pulse generator and
Fig. 4 shows a circuit diagram of the electric impulse generator.

It can be seen that in the drawings we have shown (1) the magnetic pulse generator, (2) adjustable support for the magnetic pulse generator, (3) adjustable inductor electrode, (4) fixed inductor electrode, (5) mechanism for adjusting the position of the adjustable inductor electrode, (6) base of the electro-immunisation chamber, (7) connections between the control panel and the chamber, (8) control panel, (9) insulating mattress, (10) DC power supply, (11) resistors, (12) capacitors, (13) induction source, (14) controls of the magnetic pulse generator, (15) galvanic power supply, (16) inducers and (17) controls of the electric impulse generator.

### PREFERRED IMPLEMENTATION OF THE INVENTION

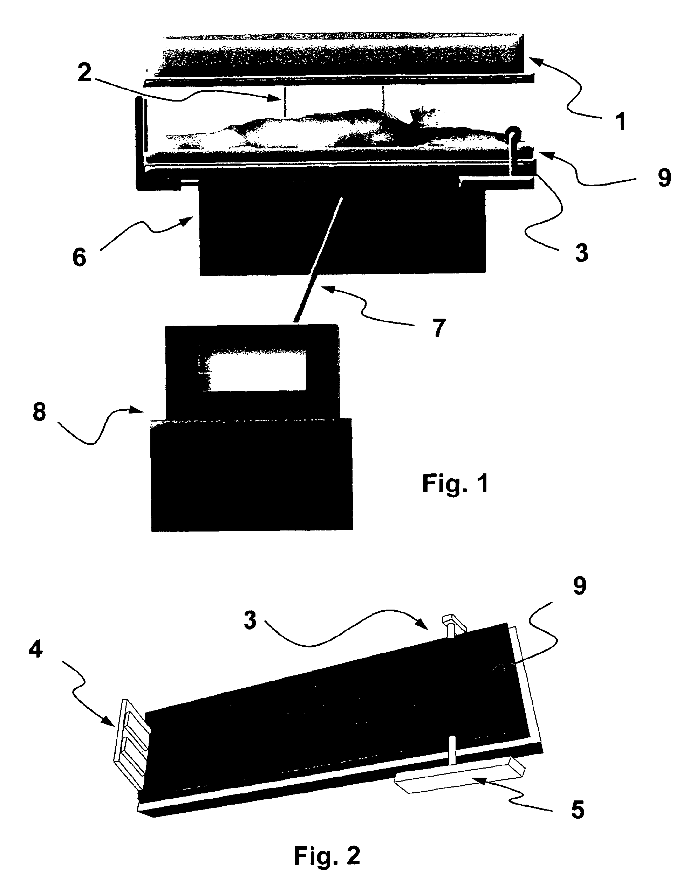

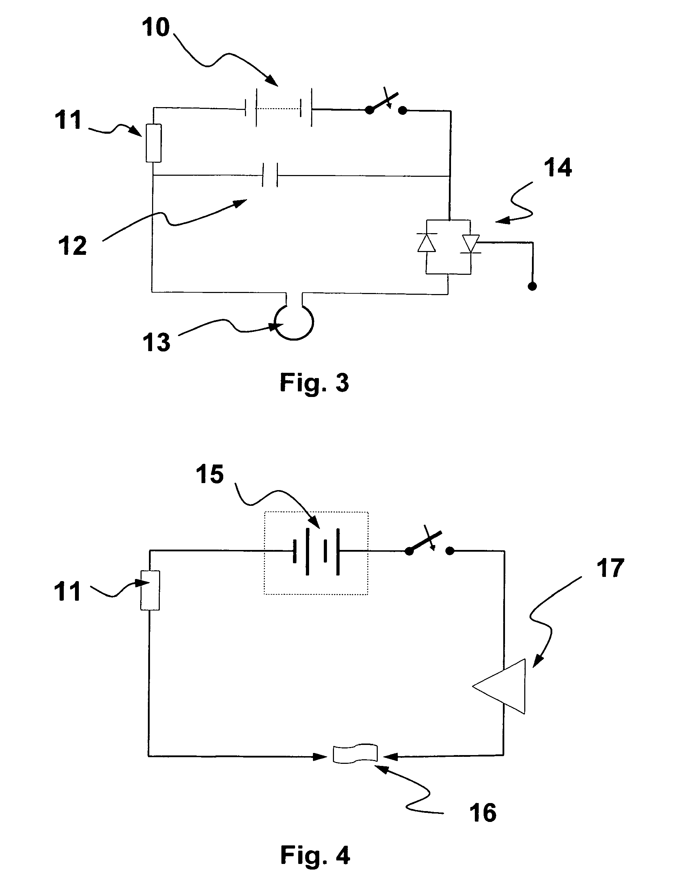

As can be seen from the drawings, the invention consists of a chamber containing the magnetic pulse generator (1) in the upper part, inside which various copper wire coils (13) are distributed, fed by the discharge from a bank of capacitors (12), which are in turn fed by a high voltage DC power supply (10) of 3,000 to 10,000 volts.
The set of coils generates a pulsing magnetic field with high penetration from its connection to a basic circuit, as seen in Fig. 3, in which, apart from the capacitors and coils already described, the total resistance (11) of the components and the control mechanisms (14) are observed, consisting of a thyristor diode which allows variation of current over time, and this diode makes it possible to circulate the current in a particular direction, and a trigger that controls the event.
This impulse generator can be adjusted in height thanks to the support (2), as can be seen in Fig. 1.

The patient is positioned in the lower area of the invention, which consists of a base (6) on which is placed an insulating mattress (9).

The electrical impulse generator is in this area, and it is made up of a rest area of insulating material on a solid base, to which two insulated electrodes are connected. These electrodes induce controlled electric stimulation in the organism of the patient.

One of the electrodes is fixed (4) and the position of the other can be adjusted based on the characteristics of the patient to be treated (3) thanks to the mechanisms (5) for adjusting the position.

The basic circuit, to which the electrodes are connected, can be seen in Fig. 4. It consists of a power supply with adjustable frequency (15), with a minimum value of 1 Hz, variable voltage from 4 to 40 Volts for work with intensity between 0 and 3 mA, as well as the total resistance in the circuit (11), control mechanisms (17) and the inductor electrodes (16).

Fig. 1 also shows the control panel (8) and the connection cables (7) between the control panel and the electro-immunisation chamber.

The invention is very simple to use, as the patient can be subjected to treatment in the electro-immunisation chamber once HIV has been diagnosed, following a suitable clinical study principally relating to use of implant devices using electronic systems in their functioning, as is the case for pacemakers.

The results are very quick, as a a patient with a viral load of 1000 cop/ml (units of HIV /ml) can reduce the quantity of virus units to less than 50 cop/ml in two treatment sessions of 15 minutes with the invention.

When electricity is induced in the medium that has been exposed to HIV within the organism, actually it is acting on the ionic channels of the GP120 by voltage. These open and lose the energy necessary for interacting with the receptors of the CD4, giving an irreversible guarantee that the viral particle cannot replicate. This interrupts the deterioration process of the immune system.

To obtain this result, a specialised galvanic power supply is used, as described in the statement, but due to the morphological structure of the human organism, it is not possible to obtain a constant potential difference to give a sufficient voltage to operate the ionic channels of the GP120 protein in HIV in all the spaces of the different organ systems. For this reason there is also a high-penetration magnetic pulse generator in the organism that provokes induced values of electricity, guaranteeing the same effect on the ionic channels of the GP120 protein present in the virus but at more complex locations, such as the lymphatic system and inside organs, where there can be reserves of the virus.

The essential points of the invention allow for variants in detail, which are also protected, and there could be changes in the type of insulating material, sources of electric power, regulation and control mechanisms; as well as the dimensions of the various parts of the system and, of course, the material used.

## Claims

1. **Electro-immunization chamber.- characterized by** the fact that it is formed by a system of devices in the form of a horizontal compartment for the therapeutic treatment of individuals diagnosed as HIV positive. On the upper surface, which is adjustable in height, there are 30 coils over an insulating base, fixed at the same height above the latter, in a spatial distribution of 250cm x 75cm, which act as a pulsating magnetic field emitting focus, where the flow value emitted is the same for each one, all attached to a controlled source which generates high voltage discharges (from 3000 V) to the same, and at the bottom, a horizontal base, to which a set of electrodes is attached, adjustable in movement at one of their ends, which guarantee a single supine decubitus position, with the upper limbs of the patient extended to make contact with these in attachment mode, and at the other end a set of electrodes fixed over the insulating surface, which guarantee direct contact with the lower limbs. Both electrode systems are attached to a galvanic current source which organizes the electric conductivity, in which the patient simulates a linear conductor.

2. **Electro-immunization chamber.-**, **characterized by** the fact that it possesses a control panel, which, using the corresponding control devices and indicators, shows the treatment exposure time, the value of the electric energy supplied to the magnetic flow generating coils in the form of discharge (from 3000v), and the value of the potential difference established between the set of electrodes connected to the galvanic source, which, because the whole system acts simultaneously, provides an energy combination described by the appearance of internal micro-current values in all the body fluid spaces of an organism, with enough energy to teach a bio-electric simulation level equal to the bio-electricity expressed by a CD4 surface receptor, being sufficient to interact with the GP120 marking molecules expressed in the virions and irreversibly subtract from the latter its capacity to identify healthy cells.
